# EUROPEAN PATENT SPECIFICATION

(11) **EP 1 943 202 B1**
(45) Date of publication and mention of the grant of the patent: **17.12.2014**
(21) Application number: 06844259.9
(22) Date of filing: 03.11.2006
(51) Int. Cl.: C07C 17/20, C07C 17/25, C07C 17/10, C07C 17/02, C07C 21/18, C07C 19/08, C07C 19/10

(54) **METHOD FOR PRODUCING FLUORINATED ORGANIC COMPOUNDS**
VERFAHREN ZUR HERSTELLUNG FLUORIERTER ORGANISCHER VERBINDUNGEN
PROCEDE DE PRODUCTION DE COMPOSES ORGANIQUES FLUORES

(30) Priority: 03.11.2005 US 733444 P; 03.11.2005 US 733383 P
(43) Date of publication of application: 16.07.2008
(60) Divisional application: 11000266.4
(73) Proprietor: Honeywell International Inc., Morristown NJ 07962 (US)
(72) Inventor: MUKHOPADHYAY, Sudip, Williamsville, NY 14221 (US); NAIR, Haridasan, Williamsville, NY 14221 (US); VAN DER PUY, Michael, Amherst, NY 14221 (US); TUNG, Hsueh Sung, Getzville, US 14068 (US); MERKEL, Daniel, C., West Seneca, US 14224 (US); DUBEY, Rajesh, Buffalo, NY 14210 (US); MA, Jing Ji, West Seneca, NY 14224 (US); LIGHT, Barbara, Niagara Falls, NY 14305 (US); FLEMING, Kim, Hamburg, NY 14075 (US); BORTZ, Cheryl, N Tonawanda, NY 14120 (US); UDY, Richard, Niagara Falls, NY 14304 (US)
(74) Representative: Hucker, Charlotte Jane
(86) International application number: PCT/US2006/042972
(87) International publication number: WO 2007/056148

(56) References cited:
- WO-A-90/08752
- GB-A- 844 597
- US-A- 2 996 555
- US-A- 6 066 769
- A. L. HENNE, F. W. HAECKL: J. AM. CHEM. SOC., vol. 63, 1941, pages 2692-2694, XP002427149
- DATABASE WPI Week 199812 Derwent Publications Ltd., London, GB; AN 1998-126110 XP002427152 & JP 10 007605 A (CENTRAL GLASS CO LTD) 13 January 1998 (1998-01-13)
- J. MARCH: "Advanced Organic Chemistry" 1977, MCGRAW-HILL INTERNATIONAL BOOK COMPANY , AUCKLAND , XP002427150 page 631 - page 636

## Description

### BACKGROUND

### (1) Field of The Invention:

This invention relates to novel methods for preparing fluorinated organic compounds, and more particularly to methods of producing fluorinated olefins.

### (2) Description of Related Art:

Hydrofluorocarbons (HFC's), in particular hydrofluoroalkenes such as tetrafluoropropenes (including 2,3,3,3-tetrafluoro-1-propene (HFO-1234yf)) have been disclosed to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFCs do not contain chlorine and thus pose no threat to the ozone layer.

Several methods of preparing hydrofluoroalkanes are known. For example, U.S. Pat. No. 4,900,874 (Ihara et al) describes a method of making fluorine containing olefins by contacting hydrogen gas with fluorinated alcohols. U.S. Pat. No. 2,931,840 (Marquis) describes a method of making fluorine containing olefins by pyrolysis of methyl chloride and tetrafluoroethylene or chlorodifluoromethane. The preparation of HFO-1234yf from trifluoroacetylacetone and sulfur tetrafluoride has been described. *See* Banks, et al., Journal of Fluorine Chemistry, Vol. 82, Iss. 2, p. 171-174 (1997). Also, U.S. Pat. No. 5,162,594 (Krespan) discloses a process wherein tetrafluoroethylene is reacted with another fluorinated ethylene in the liquid phase to produce a polyfluoroolefin product.

US-A-2996555 describes the vapour phase preparation of fluorohydrocarbons, and in particular 2,3,3,3-tetrafluoropropene (HFO-1234yf). Preparation of HFO-1234yf is described as involving vapour phase reaction of hydrogen fluoride and an organic compound having the formula CX₃CF₂CH₃, wherein X is selected from chlorine, bromine and fluorine, in a reaction zone maintained at a temperature above 150°C and containing a chromium oxyfluoride catalyst.

Henne et al J. Am. Chem. Soc. (1941) 63:2692-2694 describes the preparation of chlorinated derivatives of 2-fluoropropane, for instance by reaction of hydrogen fluoride with CH₂=CClCH₂Cl.

JP-A-10 007605 describes the preparation of 1,3,3,3-tetrafluoropropene by reaction of 1,1,1,3,3-pentachloropropane with hydrogen fluoride in the presence of a catalyst.

GB-A-844597 describes preparation of 3,3,3-trifluoropropene by contacting hydrogen fluoride and a three carbon halogenated hydrocarbon containing three or four hydrogen atoms and three or four halogen atoms other than iodine, not more than two of which are fluorine, and with one terminal carbon atom containing no hydrogen thereon, in a molar ratio of hydrogen fluoride to halogenated hydrocarbon of 3:1, for a contact time of at least 0.5 seconds and at a temperature above 150°C, and in the presence of a chromium oxyfluoride catalyst.

Notwithstanding prior teachings, applicants have come to appreciate a continuing need for methods of efficiently preparing certain hydrofluorocarbons, particularly tetrafluorpropenes such as HFO-1234yf.

### SUMMARY OF THE INVENTION

Applicants have developed a method for producing 2-chloro-3,3,3-trifluopropene (HFO-1233xf) as defined in claim 1.

In preferred embodiments, the conversion of the starting material 2-fluoro-2,3,3,3-tetrachloropropane (HCFC-241bb) in the reaction is from about 70% to about 100 %. In certain embodiments, including those which produce conversion levels as indicated herein, the selectivity of the reaction to tetrafluoropropene, and to HFO-1234yf in particular, is from about 5 to about 40%. In certain preferred embodiments the reaction product contains 2-chloro, 3,3,3-trifluoro-1-propene (HFO-1233xf) with a selectivity of from about 50% to about 90%.

### DETAILED DESCRIPTION OF PREFERRED EMBODIMENTS

One beneficial aspect of the present invention is that it enables the production of HFO-1233xf from relatively low cost and readily obtainable starting materials. More specifically, certain preferred embodiments of the present invention involve producing the desired HFO-1233xf from C3 olefin's, such as propylene. Propylene is in many embodiments an advantageous starting material because it is relatively inexpensive, is relatively easy to handle, and is generally readily available in commercial quantities.

Thus, in certain embodiments the present methods include the step of reacting C3 olefin, such as propylene, with a halogenating agent (such as ClF or HF) under conditions effective to produce CH₃CHFCH₂Cl or CH₃CHFCH₃, or to produce a compound of formula (III)

CH₃₋ₙXₙCXYCH₃ (III)

where n is 0, 1 or 2, each x is Cl, and each y is independently H or F. This reaction is sometimes referred to herein for convenience but not necessarily by way of limitation, as a halogen addition reaction.

Preferably, CH₃CHFCH₂Cl, CH₃CHFCH₃ or the compound of formula (III) is then reacted, preferably in a photo-chlorination reaction, to produce a reaction product containing at least one compound including HCFC-241bb. HCFC-241bb is then exposed to reaction conditions, which are sometimes referred to herein for convenience, but not necessarily by way of limitation, as fluorination to produce a reaction product containing HFO-1233xf. Preferred aspects of each of these preferred steps is described below, with the titles used as headings for these steps being used for convenience but not necessarily by way of limitation.

### A. REACTION OF C3 OLEFIN WITH HALOGENATING AGENT

In preferred embodiments, a compound of formula (IV)

CHₙX₃₋ₙCH=CH₂ (IV)

is contracted under reaction conditions with a compound of formula XₘY₂₋ₘ wherein X, Y are as defined above, provided that the compound is not H₂, and m is 0, 1, or 2, to produce CH₃CHFCH₂Cl, CH₃CHFCH₃ or a compound of formual (III), namely, CHₙX₃₋ₙCXYCH₃, where n is 0, 1 or 2. In preferred embodiments, the compound of formula (IV) is propylene and the compound of formula XₘY₂₋ₘ is one or more of ClF, Cl₂, F₂ and HF, preferably one or more of ClF, Cl2, and HF. The compound XₘY₂₋ₘ, and compounds which perform a similar function in the present reactions, are referred to herein as halogenation agents.

In certain preferred embodiments, the contacting step comprises contacting, (preferably by introducing into a reactor) the compounds in an halogenation agent:formula (IV) mole ratio of from about 1:1 to about 4:1, and even more preferably of from about 2:1 to about 4:1. to preferred embodiments in which the compound of XₘY₂₋ₘ comprises HF and the formula (IV) compound comprises propylene, the HF:propylene mole ratio of the feeds to the reactor are from about 1:1 to about 10:1 and even more preferably from about 2:1 to about 4:1. In preferred embodiments in which the compound of XₘY₂₋ₘ comprises ClF (X is Cl, Y is F, and m is 1) and the formula (IV) compound comprises propylene, the ClF:propylene mole ratio of the feeds to the reactor are from about 10 to about 0.1 and even more preferably from about 1 to about 0.2. In preferred embodiments in which the compound of XₘY₂₋ₘ comprises Cl₂ (m is 2 and X is Cl) and the formula (IV) compound comprises propylene, the Cl₂:propylene mole ratio of the feeds to the reactor are from about 5 to about 0.1 and even more preferably from about 1 to about 0.2.

This reaction step can be carried out in the liquid phase and/or in the gas phase, and it is contemplated that the reaction can be carried out batch wise, continuous, or a combination of these.

### 1. LIQUID PHASE HALOGEN ADDITION REACTIONS

Although not necessarily preferred, certain embodiments of the present invention (particularly when the XₘY₂₋ₘ compound is ClF, HF or Cl₂, or combinations of two or more of these) involve relatively low temperature reactions in which at least the organic reactant(s) are charged to the reactor as liquids, with the reactor preferably maintained at a temperature of from about -90°C to about -50°C, and at least a portion of the reaction is carried in the liquid phase (the normal boiling point HₙX₃₋ₙ of the preferred reactant propylene is - 47.8 °C). However, it is contemplated that at least some portion of the reaction product may be produced and/or removed from the reaction mixture in such embodiments as a gaseous material.

For those preferred embodiments which utilize ClF as a reactant, it is sometimes preferred to provide ClF, preferably in situ, by the liquid-phase reaction of HF and Cl₂ in the presence of catalyst, preferably a transition metal catalyst, and even more preferably a transition metal halide catalysts such as FeC13, TaCl5, TiCl4, SbCl5, SbCl3, and CrC13, SbF3, SbF5, AlF3, and CrF3, and combinations of two or more of these. In certain preferred embodiments, therefore, the present halogenation step comprises contacting CH3CH=CH2, HF and Cl2 in the presence of a metal catalyst, preferably a metal chloride salt, preferably with addition of kinetic energy to provide a substantially uniform reaction mixture (such as stirring), under conditions effective to form a reaction product comprising the desired compound of formula (III).

In certain preferred embodiments, the reaction is carried out at a temperature of from about -90°C to about -50°C, more preferably from about -80°C to about - 70°C, under conditions effective to achieve a percentage conversion of at least about 70%, more preferably at least about 90%, and even more preferably at least about 100% of the compound of formula (IV). Preferably, the reaction conditions are effective to achieve a percentage selectivity to compounds of formula (III), and preferably 2-fluoropropane, of at least about 50%, more preferably at least about 75%, and even more preferably at least about 95%. In certain preferred embodiments a selectivity of about 98% or greater is achieved.

As used herein, the term "percentage conversion" with respect to a reactant refers to the moles reacted in the reaction process divided by the moles of reactant in the feed to the process multiplied by 100.

As used herein, the term "percentage selectivity" with respect to an organic reaction product refers to the ratio of the moles of that reaction product to the total of the remaining organic reaction products multiplied by 100.

In certain preferred embodiments the reaction time for the preferred liquid phase halogenation reaction is from about 1 to about 3 hours. The reaction product in preferred embodiments includes one or more of CH₃CHFCH₂Cl, and/or CH₃CHFCH₃. In preferred embodiments, the reaction product comprises from about 40 wt. % to about 75 wt.% CH₃CHFCH₂Cl, and less than about 5% CH₃CHFCH₃.

It will be appreciated that many alternatives for the provision of ClF in accordance with this preferred step of the present invention are available and within the scope hereof. By way of example, the reactant ClF may be provided in certain embodiments simply by purchasing the needed quantity of the material in the appropriate form. In other preferred embodiments, it is desirable to provide the ClF by conducting a liquid-phase reaction of HF and Cl₂, preferably in the presence of transition metal halide such as SbF₅. Such reactions, especially single stage reactions, can be achieved using any equipment and conditions known and available in the art for such types of reactions, preferably at a temperature of from about -50 °C to -90°C, and even more preferably the reaction temperature is maintained at a temperature of from about -65°C to about -85°C.

In certain embodiments, a two stage reaction may be used, with the first stage being a gas phase reaction and the second stage being a liquid phase reaction. In such embodiments the ClF produced in this first reaction stage, and/or from other sources, is preferably then charged to a second reaction vessel or to a second region of the same vessel, preferably a second autoclave, where it is contacted with a compound of . formula (IV), such as propylene, at a temperature as specified above, preferably at about -75°C, and preferably under conditions capable of providing selectivity to CH₃CHFCH₂Cl or CH₃CHFCH₃, or to a compound of formula (III), preferably CH₃CHFCH₂Cl, of at least about 60% and up to about 75% or greater.

### 2. GAS/LIQUID PHASE HALOGEN ADDITION REACTIONS

Although not necessarily preferred, the formation of CH₃CHFCH₂Cl or CH₃CHFCH₃, or of a compound of formula III may also be carried out at least partially in a gas phase reaction, preferably in the presence of a catalyst. For example, high conversion and selectivity can be achieved in some embodiments by first reacting HF and Cl₂ in a liquid phase, and preferably in a continuous liquid phase reaction, by charging the reactor with a catalyst, preferably a transition metal halide such as SbF₅ and conducting the reaction at a temperature of from about -50 °C to 50°C to produce C1F. In such embodiments it is preferred to introduce the ClF produced in the gas phase, together with a compound of formula (IV), into a liquid phase reactor, generally in accordance with the conditions described above, to produce CH₃CHFCH₂Cl or CH₃CHFCH₃, or a compound of formula (III), preferably at a selectivity to CH₃CHFCH₂CI of at least about 60%, and even more preferably at least about 70%.

### 3. PREFERRED GAS PHASE HALOGENATION REACTIONS

For certain preferred embodiments, particularly those preferred embodiments which utilize HF as a reactant, it is preferred to provide CH₃CHFCH₂Cl or CH₃CHFCH₃, or a compound of formula (III) by a gas-phase reaction of HF and a compound of formula (IV) in the presence of catalyst, preferably a transition metal catalyst, and even more preferably a transition metal halide catalysts such as FeCl₃, TaCl₅, TiCl₄, SbCl₅, SbCl₃, and CrCl₃, SbF₃, SbF₅, AlF₃, and CrF₃, and combinations of two or more of these. In certain preferred embodiments, therefore, the preferred halogenation step comprises contacting CH₃CH=CH₂, preferably an iron based catalyst, and even more preferable an iron chloride catalyst, and maintaining the reactor temperature at from about 20°C to about 100°C, more preferably from about 25°C to about 35°C, with a preferred reactor pressure of from about 103 kPa (15 psia) to about 1379 kPa (200 psia), and even more preferably from about 138 kPa (20 psia) to about 690 kPa (100 psia). In such preferred embodiments HF is preferably charged to the reactor, preferably as a liquid (for example by maintiaing the vessel containing the HF under pressure, such as 310 kPa gauge (45 psig) of N₂ head space pressure) and the compound of formula (IV) is charged to the reactor, preferably as a gas. In such embodiments the conversion of the formula (IV) compound, particularly propylene, is preferably at least about 40%, more preferably at least about 80%, and selectivity to CH₃CHFCH₂Cl or CH₃CHFCH₃, or to compounds of formula (III), particularly 2-fluoropropane, is preferably at least about 40%, more preferably at least about 60%, more preferably at least about 80%, and in certain embodiments at least about 90%. In certain preferred embodiments selectivity of about 95% is preferred. Examples of other catalysts that may be used in this aspect of the invention include carbon, carbon nanotubes, SbCl₅C, SbF₅/C, FeCI₃, CrF₃, Cr-oxyfluoride, and Cr₂03.

### B. CHLORINATION OF CH₃CHFCH₂Cl, CH₃CHFCH₃, or THE COMPOUND OF FORMULA III

According to preferred aspects of this invention, CH₃CHFCH₂Cl or CH₃CHFCH₃, or a compound of formula (III), preferably but not necessarily produced in accordance with the procedures described above, is chlorinated to produce HCFC-241bb. In its broad aspects, the preferred chlorination step is amenable to a large number of specific processing condition and steps in accordance with the teachings contained herein, and all such variations are within the broad scope of the present invention. It is particularly preferred, however, that the chlorination step comprise photochlorination. Thus, the preferred reaction step comprises exposing the reactants, preferably in a liquid phase, to ultraviolet radiation, preferably in the range of from about 200 to about 400nm. The chlorination agent preferably comprises chlorine gas, either neat or preferably with a diluent such as nitrogen. A chorination catalyst, such as V₂O₅, Zeolites, and Au/Ti0₂ catalyst, may be used in certain embodiments. The reaction is preferably carried out a temperature of from about -20°C to about 200°C, and even more preferably from about 25°C to about 120°C for a time of from about 5 seconds to about 5 hours, more preferably from about 15 seconds to about 30 min. The reaction product, which comprises a compound of formula (I), may then optionally be subject to one or more separation steps, such as distillation, to remove unwanted byproducts and produce a stream relatively concentrated in compounds of the formula (I).

### C. FORMATION OF HFO-1233xf

The methods of the present invention preferably comprise reacting HCFC-241bb with a fluorinating agent to produce HFO-1233xf.

It is contemplated that this reaction step may be preformed using a wide variety of process parameters and process conditions in view of the overall teachings contained herein. This reaction step comprise a gas phase catalyzed reaction, wherein the catalyst is selected from FeCl₃, chromiumoxyfluoride, Ni (including Ni mesh), NiCl₂, CrF₃. Other catalysts include carbon-based catalysts, antimony-based catalysts (such as SbCl₅), aluminum-based catalyst (such as AlF₃ and Al₂O₃). Many other catalysts may be used, including palladium-based catalyst, platinum-based catalysts, Rhodium-based catalysts and ruthenium-based catalysts. Of course, two or more any of these catalysts, or other catalysts not named here, may be used in combination.

In general it is preferred that the catalysts are pretreated by fluorination prior to use. In preferred embodiments, fluorination of the catalysts comprises exposing the catalyst to a stream of HF at about reaction temperature and pressure.

In certain preferred embodiments, the present step of fluorinating HCFC-241bb to produce HFO-1233xf comprises contacting HCFC-241bb with a fluorinating agent, preferably under conditions effective to provide a formula I conversion of at least about 10%, more preferably at least about 55%, and even more preferably at least about 70%. In certain preferred embodiments the conversion is at least about 90%, and more preferably about 100%. Furthermore, in certain preferred embodiments, the present step of fluorinating HCFC-241bb to produce HFO-1233xf is conducted under conditions effective to provide a HFO-1233xf selectivity of at least about 5%, more preferably at least about 20%, more preferably at least about 50%, and even more preferably at least about 90%. The selectivity of the reaction to HFO-1234yf is at least about 5%, more preferably at least about 10%, more preferably at least about 50% or higher, and the selectivity to HFO-1233xf is at least about 5%, more preferably from about 30% to about 70%, and even more preferably from about 40% to about 60%.

### EXAMPLES

Additional features of the present invention are provided in the following examples, which should not be construed as limiting the claims in any way.

### Examples 1 - 24:

These examples illustrate gas phase fluorination-dehydrohalogenation of CCl₃CFClCH₃ (241bb) to CF₃CCl=CH₂ (1233xf) and CF₃CF=CH₂(1234yf). A 56cm (22-inch), 1.3 cm (1/2-inch) diameter Monel tube reactor is charged with 120 cc of catalyst, as specified in Table I below. All catalysts are fluorinated for 6 h with 80 glh of HF at reaction temperature under (138 kPa gauge (20 psig) pressure. The reactor is mounted inside a heater with three zones (top, middle and bottom). The reactor temperature is monitored using a 5-point thermocouples kept at the middle inside of the reactor. The inlet of the reactor is connected to a pre-heater, which is kept at about 300°C by electrical heating. Liquid HF is fed from a cylinder into the pre-heater through a needle valve, liquid mass-flow meter, and a control valve at a constant flow of 1-1000 g/h. The HF cylinder is kept at a constant pressure of about 276 kPa gauge (40 psig) by applying anhydrous N₂ gas pressure into the cylinder head space. 10-120 g/h of organic compound of formula I (HCFC-241bb) is fed from a cylinder kept at about 145°C through a regulator, needle valve, and a gas mass-flow-meter. The compound of formula I is also fed time to time as liquid at 120°C from a cylinder into the pre-heater through a needle valve, liquid mass-flow meter, and a control valve at a constant flow of 50-150 g/h. The organic line from the cylinder to the pre-heater is kept at 265°C by wrapping with constant temperature heat trace and electrical heating. All feed cylinders are mounted on scales to monitor their weight by difference. The reactions are run at a constant reactor pressure of about 0-690 kPa gauge (0-100 psig) by controlling the flow of reactor exit gases by another control valve. The gases exiting the reactor are analyzed by on-line GC and GC/MS connected through a hotbox valve arrangement to prevent condensation. The product was collected by flowing the reactor exit gases through a 20-60 wt% aq. KOH scrubber solution and then trapping the exit gases from the scrubber into a cylinder kept in dry ice or liquid N₂. The products were then isolated by distillation. The results are shown in Table I below.

**Table 1: CCl₃CFClCH₃ + 3HF → CF₃CF=CH₂ + HCl**

| Example# / Catalyst | T, C | % Conversion of 241bb | % Selectivity to 1234yf | % Selectivity to 1233xf | % Selectivity 1234yf/1233xf |
|---|---|---|---|---|---|
| Example 1/ Chromium Oxoxyfluoride | 250 | 56 | 1 | 12 | 0.08 |
| Example 2/ (Plant Catalyst) | 320 | 92 | 7 | 85 | 0.08 |
| Example 3/ (Plant Catalyst) | 350 | 96 | 8 | 69 | 0.11 |
| Example 4/ (Plant Catalyst) | 375 | 100 | 12 | 63 | 0.19 |
| Example 5/ 4-6wt%FeCl3/C | 50 | Plugged | | | |
| Example 6/ (NORIT-RFC-3) | 100 | Plugged | | | |
| Example 7 | 120 | 100 | 14 | 57 | 0.25 |
| Example 8 | 150 | 100 | 14 | 61 | 0.23 |
| Example 9 | 200 | 100 | 11 | 54 | 0.2 |
| Example 10 | 220 | 100 | 9 | 43 | 0.2 |
| Example 11/ Carbon | 300 | 56 | 11 | 26 | 0.42 |
| Example 12/ Carbon | 400 | 71 | 9 | 17 | 0.53 |
| Example 13/ Carbon | 500 | 89 | 5 | 14 | 0.36 |
| Example 14/ Ni-mesh | 250 | 42 | 7 | 29 | 0.24 |
| Example 15/ Ni-mesh | 350 | 84 | 10 | 62 | 0.16 |
| Example 16/ Ni-mesh | 425 | 100 | 14 | 53 | 0.26 |
| Example 17/ 25 wt% SbCls/C | 110@ 345 kPa gauge (50 psig) | 100 | 18 | 59 | 0.3 |
| Example 18/ 25 wt% SbCls/C | 120@ 345 kPa gauge (50 psig) | 100 | .21 | 49 | 0.42 |
| Example 19/ 1.4wt% NiCl2/Al2O3 | 250 | | | | |
| Example 20/ 1.4wt% NiCl2/Al2O3 | 320 | 92 | 14 | 62 | 022 |
| Example 21/ 1.4wt% NiCl2/Al2O3 | 350 | | | | |
| Example 22/ 1.4wt%Ni/Al203 | 350 | 100 | 6 | 52 | 0.12 |
| Example 23/ AlF3 | 150 | 82 | 0 | 8 | 0 |
| Example 24/ CrF3 | 200 | 89 | 2 | 11 | 0.18 |

In addition to the products identified in the table, other compounds produced in the reaction are 1,1,1-trifluoroethane (143a), 3-chloro-2,2,3,3-tetrafluoropropane(244cc), 3,3-dichloro-2,2,3-trifluoropropane(243cc), 1-propene-1,1-dichloro-2-fluoro, 2,3,3-trichloro-2-fluoropropane(251bb), 1-chloro-3,3,3-trifluoro-1-propene (1233zd), 3,3,3-trifluoro-1-propene (1243zf),1,1-dichloro-2-fluoro4-propene,2,3,3-trichloro-2-fluoropropane, dichlorodifluoropropene, and carbon black.

### Comparative Example 25

This example illustrates addition of F₂ to CH₃CH=CH₂ in a liquid phase reaction, which is illustrated by the following reaction scheme:

CH₃CHCH₂ + F₂ → CH₃CHFCH₂F

About 1 - 100 wt% F₂ in nitrogen is bubbled through 125 g of liquid propylene in a stirred Hastrelloy C reactor at about -50°C to -75°C for about 1 hour in the presence of HF as the solvent. Al gallon Parr reactor is first charged with a relatively inert solvent, HF, to help with heat transfer and dilution of the organic. Then 125 grams of propylene are added batch wise to the reactor. The reaction mixture is continuously mixed and cooled to the desired temperature. Then the F₂ feed (1 wt%), diluted with N2 (99 wt%), is introduced continuously directly into the reaction mixture through a dip tube until about 90% of the stoichiometric amount needed to convert all the propylene that is added. The reactor temperature and pressure are controlled automatically at the desired set points of between -50 to -75°C and a constant pressure of 40 psig. The temperatures are chosen to minimize the amount of propylene that would exit the reactor with the N₂ diluent. The gases exiting the reactor are passed through a caustic scrubber carboy and an activated alumina column to remove acidity, then a dri-rite column to remove moisture, and finally the organic is collected in a DIT. When the desired amount of F₂ is added the reaction liquid is sampled. The sample is absorbed in H₂O and the organic is recovered by phase separation. The organic is then analyzed by GC and GC/MS. The remaining material in the reactor is boiled off through the scrubbing system and the organic is collected in the DIT and analyzed by GC and GC/MS. The analyses are used to determine that the reaction has an overall selectivity to CH₃CHFCH₂F of about 36-45%.

### Comparative Example 26

This example illustrates addition of F₂ to CH₃CH=CH₂ in a gas phase reaction, which is illustrated by the following reaction scheme:

CH₃CHCH₂ + F₂→ CH₃CHFCH₂F

The same apparatus as described in Example 25 is used, except that gaseous propylene and 1% F2 (99% dilution w/ N2) are fed into the Parr reactor via a common dip tube. Propylene is fed at a 50% stoichiometric excess. The reactor is kept at 70°C and at atmospheric pressure. The reactor effluent is passed through a D1T, which collected most of the organic. Only a couple of grams of vapor are left in the Parr reactor at the end of the experiment GC analysis of the material indicated about 10% conversion of the propylene. The selectivity to CH₃CHFCH₂F is about 32% based on GC area%.

### Comparative Example 27

This example illustrates addition of F2 to CH3CH=CH2 in a gas phase reaction under the same conditions as Example 26, except the reaction is performed at higher temperature of about 0 to -20°C. A yield of about 10% CF₃CHFCH₂F is obtained, which is then transformed to HFO-1234yf with 100% selectivity in the next step by reacting with 20%KOH solution in the presence of 18-crown ether as the phase-transfer catalyst at about 55°C using a 300 ml autoclave.

### Example 28

This example illustrates addition of fluorine to CH₃CH=CH₂ in a gas phase reaction, which is illustrated by the following reaction scheme:

CH₃CHCH₂ + HF → CH₃CHFCH₃

In a gas-phase reaction a Monel tube reactor was charged with 1909 cc of 4-6-wt% FeCl₃/C (NORIT-RFC 3, commercial catalyst from NORIT NEDERLAND B.V.). The reactor was mounted in a constant temperature sand-bath with 40-50 ml/min of air flowing through the sand-bath. The sand-bath set point was set at 28°C during the reaction. The inlet of the reactor was connected to a pre-heater which was kept at 106°C by supplying 207 kPa gauge (30 psig) steam through the jacket. The liquid-HF was fed from a cylinder into the pre-heater through a positive displacement pump and kept at a constant flow of 15.88 mol/h (318 g/h) by controlling the flow with a research control valve. The HF cylinder was kept at a constant pressure of 207-276 kPa gauge (30-40 psig) by applying anhydrous N₂ gas pressure into the cylinder head space. A 5.4 mol/h (227 g/h) of propylene is fed as a gas from a cylinder through a regulator, needle valve, and flow controller directly into the reactor inlet at a point just after the pre-heater. HF and Propylene cylinders were mounted on two different scales to monitor their weight by difference. The reactions were run at a constant reactor pressure of 172 kPa gauge (25 psig) by controlling the flow of reactor exit gases by another research control valve. The mole ratio of HF to Propylene was kept at 2.94 with a contact time of 33 sec. The exit gases coming out of the reactor were analyzed by an on-line GC and GCMS connected through a hotbox valve arrangements to prevent condensation. The conversion of Propylene was almost 100% and the selectivity to 2-Fluoropropane was 98%. The reaction was performed continuously over 4 days period and the catalyst did not loose any activity. The product was collected by flowing the reactor exit gases through a 20-60 wt% aq. KOH scrubber solution and then trapping them in a cylinder kept inside dry ice or liquid N₂.

### Examples 29 - 42

This example illustrates the chlorination of 2-fluoropropane (CH₃CHFCH3) to CCl3CFClCH3 (241bb) in a gas phase reaction, which is illustrated by the following reaction scheme:

CH₃CHFCH₃ + 4Cl_{2 → (light)} CCl₃CFClCH₃ + 4HCl

A 500 ml Pyrex reactor was placed inside a Rayonet photo chlorinator at room temperature (25°C). The reactor used in this study was a RAYONETModel-RPR-100 with standard operating temperature of 35°C and standard power consumption around 400 watts. The inside of the photo chlorinator was equipped with 16 UV-lamps; the number of lamps could be varied easily. The 2-Fluoropropane was fed into the reactor from a cylinder kept at a constant water bath temperature of 35°C through a regulator and mass-flowcontroller. Chlorine gas was fed from a cylinder through a regulator, needle valve, and a massflow-controller into the reactor. N₂ was used as the diluents, though the reaction proceeds well without it from a cylinder, which was fed from a cylinder through regulator and mass-flowcontroller. A fan at the bottom of the photo chlorinator was kept on with a constant flow of N₂ to helped keeping the reactor at a constant temperature. This was because of fast dissipation of heat of reaction to avoid run-away reaction. The products that were high boiling liquids stayed inside the reactor collection section, collected and analyzed after the stipulated reaction time. The exit gases out of the reactor was analyzed by on-line GC which contains mainly unreacted 2-fluoropropane, N2 and mono-chlorinated products. The liquid products thus obtained was then washed with water to remove HCl and soluble Chlorine and then purified by distillation under vacuum. The experimental mp of 241bb is 98-101°C and boiling is 139°C. The purity was around 95-100%. The results are shown in Table 2 below.

**Table 2 - Photo chlorination of 2-Fluoropopane (R281ea) to CCl₃CFClCH₃ (241bb)**

| Ex # | FP, Sccm | Cl₂, sccm | N₂, sccm | Contact time, sec | Mole ratio of Cl₂ toFP | T, h | Lamp | %Conversion | %Sel. to CCl₃CF CICH₃ | %Overall sel. |
|---|---|---|---|---|---|---|---|---|---|---|
| 29 | 20 | 96 | 0 | 206 | 4.8 | 2 | 8 | 48 | 18 | 49 |
| 30 | 20 | 80 | 0 | 239 | 4 | 2 | 8 | 52 | 29 | 62 |
| 31 | 20 | 80 | 50 | 159 | 4 | 1 | 8 | 54 | 38 | 71 |
| 32 | 20 | 80 | 100 | 120 | 4 | 1 | 8 | 46 | 19 | 63 |
| 33 | 20 | 120 | 50 | 126 | 6 | 1 | 8 | 75 | 35 | 69 |
| 34 | 20 | 150 | 50 | 108 | 7.5 | 1 | 8 | 34 | 19 | 81 |
| 35 | 20 | 20 | 76 | 206 | 1 | 1 | 8 | 27 | 26 | 85 |
| 36 | 20 | 40 | 56 | 206 | 2 | 1 | 8 | 35 | 20 | 81 |
| 37 | 20 | 60 | 36 | 206 | 3 | 1 | 8 | 35 | 28 | 82 |
| 38 | 20 | 80 | 16 | 206 | 4 | 1 | 8 | 42 | 32 | 74 |
| 39 | 20 | 96 | 0 | 206 | 4.8 | 1 | 8 | 50 | 42 | 73 |
| 40 | 20 | 96 | 0 | 206 | 4.8 | 0.25 | 8 | 45 | 35 | 70 |
| 41 | 20 | 96 | 0 | 206 | 4.8 | 0.5 | 8 | 40 | 37 | 76 |
| 42 | 20 | 96 | 0 | 206 | 4.8 | 0.75 | 8 | 54 | 41 | 71 |

| | | | | | | | | | | |
|---|---|---|---|---|---|---|---|---|---|---|
| *FP is 2-Fluoropropane; sccm is standard cubic centimeter per minute; total volume of the reactor in contract with the light is 450 ml (Pyrex); Conversion is the ratio of moles of FP converted to products to total moles of FP fed; Selectivity to CCl₃CFClCH₃ is the ratio of moles of FP converted to CCl₃CFClCH₃ to total moles of FP reacted; Overall selectivity is the ratio of moles of FP converted to CH₃CFCICH₃, CICH₂CFCICH3, Cl₂CHCFCICH3, and CCl₃CFClCH₃ to total moles of FP reacted. There are total 16 lamps. The reactor is a BAYONET-Model-RPR-100, standard operating temperature 35°C and standard power consumption 400 watts.* | | | | | | | | | | |

### Examples 43 - 72

This example illustrates the photochlorination of 2-fluoropropane (CH₃CHFCH₃) to CCl₃CFClCH₃ (241bb) in a gas phase reaction as described in Examples 29 - 42, except 4 parameters are varied, including flow rates of Cl₂ and 2-Fluoropropane, time of irradiation, and number of lamps at room temperature. The results are reported in Table 3 below:

**TABLE 3 - Photo chlorination of 2•Fluoropopane to CCl₃CFClCH₃**

| Ex # | Cl₂, Sccm | 2-FP, sccm | Time, min | No of lamps | N₂, sccm | Contact time, sec | Mole ratio of Cl₂ to 2-FP | % 2-FP Conversion | %Sel. to CCl₃CF ClCH₃ | %Overall sel. to mono-, di-, tri-and tetra-chloro compound S |
|---|---|---|---|---|---|---|---|---|---|---|
| 43 | 80 | 40 | 20 | 4 | 25 | 165 | 2 | 65 | 57 | 68 |
| 44 | 80 | 20 | 20 | 4 | 25 | 191 | 4 | 70 | 44 | 74 |
| 45 | 50 | 30 | 30 | 6 | 25 | 228 | 1.7 | 31 | 54 | 67 |
| 46 | 50 | 30 | 30 | 6 | 25 | 228 | 1.7 | 31 | 54 | 67 |
| 47 | 20 | 20 | 20 | 4 | 25 | 367 | 1 | 54 | 24 | 90 |
| 48 | 20 | 40 | 20 | 8 | 25 | 281 | 0.5 | 34 | 14 | 87 |
| 49 | 20 | 40 | 40 | 4 | 25 | 281 | 0.5 | 33 | 18 | 93 |
| 50 | 80 | 40 | 40 | 8 | 25 | 281 | 0.5 | 54 | 48 | 57 |
| 51 | 80 | 40 | 20 | 8 | 25 | 165 | 2 | 100 | 55 | 61 |
| 52 | 20 | 40 | 20 | 4 | 25 | 281 | 0.5 | 22 | 1.8 | 78 |
| 53 | 80 | 20 | 40 | 4 | 25 | 191 | 4 | 72 | 50 | 57 |
| 54 | 80 | 20 | 20 | 8 | 25 | 191 | 4 | 96 | 28 | 43 |
| 55 | 80 | 40 | 40 | 4 | 25 | 164 | 2 | 55 | 55 | 64 |
| 56 | 80 | 20 | 40 | 8 | 25 | 191 | 4 | 82 | 29 | 38 |
| 57 | 20 | 40 | 40 | 8 | 25 | 281 | 0.5 | 26 | 14 | 81 |
| 58 | 20 | 20 | 40 | 4 | 25 | 368 | 1 | 47 | 15 | 85 |
| 59 | 50 | 30 | 30 | 6 | 25 | 228 | 1.7 | 37 | 49 | 62 |
| 60 | 50 | 30 | 30 | 6 | 25 | 228 | 1.7 | 37 | 49 | 62 |
| 61 | 20 | 20 | 40 | 8 | 25 | 368 | 1 | 58 | 30 | 68 |
| 62 | 20 | 20 | 20 | 8 | 25 | 368 | 1 | 34 | 32 | 74 |
| 63 | 50 | 30 | 10 | 6 | 25 | 228 | 1.7 | 90 | 48 | 67 |
| 64 | 110 | 30 | 30 | 6 | 25 | 145 | 3.7 | 61 | 52 | 63 |
| 65 | 50 | 50 | 30 | 6 | 25 | 191 | 1 | 30 | 54 | 67 |
| 66 | 50 | 10 | 30 | 6 | 25 | 281 | 5 | 100 | 3 | 27 |
| 67 | 0 | 30 | 30 | 6 | 25 | 531 | 0 | 0 | 0 | 0 |
| 68 | 50 | 30 | 30 | 6 | 25 | 228 | 1.7 | 31 | 54 | 67 |
| 69 | 50 | 30 | 30 | 6 | 25 | 228 | 1.7 | 31 | 54 | 67 |
| 70 | 50 | 30 | 30 | 10 | 25 | 228 | 1.7 | 58 | 44 | 52 |
| 71 | 50 | 30 | 30 | 2 | 25 | 228 | 1.7 | 68 | 56 | 77 |
| 72 | 50 | 30 | 50 | 6 | 25 | 228 | 1.7 | 63 | 54 | 64 |

## Claims

1. A method of preparing 2-chloro-3,3,3-trifluoropropene (HFO-1233xf) comprising contacting hydrogen fluoride with 2-fluoro-2,3,3,3-tetrachloropropane (HCFC-241bb), said contacting step being carried out in the gas phase and in the presence of a catalyst selected from FeCl₃, chromiumoxyfluoride, Ni, Ni mesh, NiCl₂, CrF₃, carbon-based catalysts, antimony-based catalysts, aluminum-based catalysts, palladium-based catalysts, platinum-based catalysts, rhodium-based catalysts, ruthenium-based catalysts and combinations thereof.

2. A method according to claim 1, wherein the catalyst comprises an aluminium - based catalyst selected from AlF₃ and Al₂O₃.

3. A method according to claim 1, wherein the catalyst comprises SbCl₅.

4. The method of claim 1 wherein said HCFC-241bb is formed by a chlorinating step comprising contacting 2-fluoropropane with a chlorinating agent.

5. The method of claim 4 wherein said chlorinating agent comprises chlorine gas.

6. The method of claim 4 or claim 5 wherein said chlorinating step is conducted in the presence of light having a wavelength of from 200 to 400 angstroms.

7. The method of any of claims 4 to 6 wherein said chlorinating step is carried out at a temperature of from 25°C to 120°C for a time of from 5 seconds to 5 hours.

8. The method of any of claims 4 to 7 wherein said 2-fluoropropane is formed by contacting propylene with HF.

## Patentansprüche

1. Verfahren zur Herstellung von 2-Chlor-3,3,3-trifluorpropen (HFO-1233xf), bei dem man Fluorwasserstoff mit 2-Fluor-2,3,3,3-tetrachlorpropan (H-FCKW 241bb) kontaktiert, wobei der Kontaktierungsschritt in der Gasphase und in Gegenwart eines aus FeCl₃, Chromoxidfluorid, Ni, Ni-Netz, NiCl₂, CrF₃, auf Kohlenstoff basierenden Katalysatoren, auf Antimon basierenden Katalysatoren, auf Aluminium basierenden Katalysatoren, auf Palladium basierenden Katalysatoren, auf Platin basierenden Katalysatoren, auf Rhodium basierenden Katalysatoren, auf Ruthenium basierenden Katalysatoren und Kombinationen davon ausgewählten Katalysators durchgeführt wird.

2. Verfahren nach Anspruch 1, bei dem der Katalysator einen auf Aluminium basierenden Katalysator, der aus AlF₃ und Al₂O₃ ausgewählt ist, umfasst.

3. Verfahren nach Anspruch 1, bei dem der Katalysator SbCl₅ umfasst.

4. Verfahren nach Anspruch 1, bei dem das H-FCKW 241bb durch einen Chlorierungsschritt gebildet wird, bei dem man 2-Fluorpropan mit einem Chlorierungsmittel kontaktiert.

5. Verfahren nach Anspruch 4, bei dem das Chlorierungsmittel Chlorgas umfasst.

6. Verfahren nach Anspruch 4 oder Anspruch 5, bei dem der Chlorierungsschritt in Gegenwart von Licht mit einer Wellenlänge von 200 bis 400 Ängström durchgeführt wird.

7. Verfahren nach einem der Ansprüche 4 bis 6, bei dem der Chlorierungsschritt bei einer Temperatur von 25°C bis 120°C über einen Zeitraum von 5 Sekunden bis 5 Stunden durchgeführt wird.

8. Verfahren nach einem der Ansprüche 4 bis 7, bei dem das 2-Fluorpropan durch Kontaktieren von Propylen mit HF gebildet wird.

## Revendications

1. Méthode de préparation de 2-chloro-3,3,3-trifluoropropène (HFO-1233xf), comprenant la mise en contact de fluorure d'hydrogène avec du 2-fluoro-2,3,3,3-tétrachloropropane (HCFC-241bb), ladite étape de mise en contact étant réalisée en phase gazeuse et en présence d'un catalyseur choisi parmi FeCl₃, l'oxyfluorure de chrome, Ni, un mesh de Ni, NiCl₂, CrF₃, les catalyseurs à base de carbone, les catalyseurs à base d'antimoine, les catalyseurs à base d'aluminium, les catalyseurs à base de palladium, les catalyseurs à base de platine, les catalyseurs à base de rhodium, les catalyseurs à base de ruthénium, et des combinaisons de ceux-ci.

2. Méthode selon la revendication 1, dans laquelle le catalyseur comprend un catalyseur à base d'aluminium choisi parmi AlF₃ et Al₂O₃.

3. Méthode selon la revendication 1, dans laquelle le catalyseur comprend du SbCl₅.

4. Méthode selon la revendication 1, dans laquelle ledit HCFC-241bb est formé par une étape de chloration comprenant la mise en contact de 2-fluoropropane avec un agent de chloration.

5. Méthode selon la revendication 4, dans laquelle ledit agent de chloration comprend du chlore gazeux.

6. Méthode selon la revendication 4 ou la revendication 5, dans laquelle ladite étape de chloration est effectuée en présence de lumière ayant une longueur d'onde allant de 200 à 400 Angströms.

7. Méthode selon l'une quelconque des revendications 4 à 6, dans laquelle ladite étape de chloration est effectuée à une température allant de 25°C à 120°C pendant une période allant de 5 secondes à 5 heures.

8. Méthode selon l'une quelconque des revendications 4 à 7, dans laquelle ledit 2-fluoropropane est formé par la mise en contact de propylène avec du HF.
